(19) Europäisches Patentamt
European Patent Office
Office européen des brevets

(11)  **EP 1 896 329 B2**

(12)  **NOUVEAU FASCICULE DE BREVET EUROPEEN**
Après la procédure d'opposition

(45) Date de publication et mention de la décision concernant l'opposition:
**22.11.2017 Bulletin 2017/47**

(45) Mention de la délivrance du brevet:
**24.03.2010 Bulletin 2010/12**

(21) Numéro de dépôt: 06777287.1

(22) Date de dépôt: **08.06.2006**

(51) Int Cl.:
**B65B 55/10** *(2006.01)*  **B65B 3/02** *(2006.01)*

(86) Numéro de dépôt international:
**PCT/EP2006/063002**

(87) Numéro de publication internationale:
**WO 2006/136498 (28.12.2006 Gazette 2006/52)**

(54) **INSTALLATION PRODUISANT DES BOUTEILLES STERILES PAR SOUFFLAGE A PARTIR DE PREFORMES STERILISEES**

VORRICHTUNG ZUM BLASFORMEN VON STERILEN FLASCHEN AUS STERILISIERTEN VORFORMLINGEN.

INSTALLATION FOR PRODUCING STERILE BOTTLES BY BLOW MOLDING STERILIZED PREFORMS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.06.2005 FR 0551751**

(43) Date de publication de la demande:
**12.03.2008 Bulletin 2008/11**

(73) Titulaire: **Sidel Participations**
**76930 Octeville Sur Mer (FR)**

(72) Inventeurs:
• **ADRIANSENS, Eric**
  **76930 Octeville-sur-Mer (FR)**
• **HEBERT, Stéphane**
  **76930 Octeville-sur-Mer (FR)**

(74) Mandataire: **Grassin d'Alphonse, Emmanuel Jean Marie**
**Sidel Participations**
**Avenue de la Patrouille de France**
**76930 Octeville-sur-mer (FR)**

(56) Documents cités:
| | |
|---|---|
| EP-A- 0 894 543 | EP-A- 1 056 481 |
| EP-A2- 0 411 377 | DE-A1- 3 301 525 |
| DE-A1- 3 324 825 | DE-A1- 4 305 478 |
| FR- - 991 641 | JP-A- 03/ 226 444 |
| US-B1- 6 562 281 | US-B1- 6 692 684 |

**EP 1 896 329 B2**

## Description

L'invention concerne une installation produisant des bouteilles stériles, par soufflage à partir de préformes en matière plastique.

L'invention concerne plus particulièrement une installation produisant des bouteilles stériles par soufflage à partir de préformes en matière plastique, du type dans lequel les préformes sont convoyées à l'intérieur de l'installation selon un flux continu qui circule de l'amont vers l'aval, et du type comportant :

- une unité de traitement stérilisant comportant un dispositif de brumisation pourvu d'au moins une buse qui projette, vers le col de chaque préforme en cours de traitement, un flux de produit stérilisant, sous forme d'un jet de vapeur, en vue de stériliser au moins les parois internes de la préforme,
- une unité de conditionnement thermique comportant au moins un four qui chauffe les préformes,
- une unité de moulage comportant au moins un moule et au moins un dispositif de soufflage qui soumet chaque préforme à une surpression interne de manière qu'elle prenne la forme de l'empreinte du moule ce qui produit une bouteille.

Le document US-A-2001/0010145 décrit un exemple d'une installation de ce type destinée à produire des bouteilles stériles par soufflage à partir de préformes stérilisées.

Ce type d'installation présente l'inconvénient de nécessiter un débit de produit stérilisant et/ou une pression d'injection du produit stérilisant de valeurs importantes pour réussir à couvrir complètement les parois internes des préformes de manière à stériliser complètement l'intérieur des préformes.

Par conséquent, la consommation de l'installation en produit stérilisant est donc importante et l'opération de stérilisation coûteuse.

Un tel type d'installation est plus particulièrement décrit et représenté dans le document WO-A2-99/03667 qui concerne un procédé et une installation pour la fabrication de récipients stériles en matière plastique.

L'installation comporte un dispositif d'amenée des préformes à des moyens de chauffage en amont duquel les préformes sont traitées, au moins à l'intérieur des préformes, par des moyens de stérilisation. Les moyens de stérilisation comprennent notamment un produit de stérilisation, tel qu'une solution chimique de peroxyde d'hydrogène (H$_2$O$_2$) activable thermiquement, notamment par la chaleur des moyens de chauffage.

Les moyens de stérilisation comportent pour ce faire un pulvérisateur constitué par un pistolet de vaporisation qui permet de mouiller l'intérieur des préformes avec du produit de stérilisation "froid", c'est-à-dire n'ayant pas été préalablement chauffé et se trouvant à l'état liquide.

Ensuite, le produit de stérilisation va, sous l'effet de la chaleur, être activé avant de s'évaporer.

Dans l'installation décrite dans ce document, le pistolet est typiquement un pistolet dit de type bi-fluide.

En effet, le pistolet de vaporisation comporte une buse de liquide et une buse d'air formant un ensemble de projection circulaire qui est susceptible d'être placé au-dessus du trajet des préformes de manière à pulvériser un brouillard de produit de stérilisation.

Le brouillard de produit de stérilisation est formé d'un nuage de gouttelettes qui est projeté par le pistolet de vaporisation vers l'intérieur de la préforme suivant un écoulement de type turbulent.

Par définition, un écoulement est dit "turbulent" lorsque le nombre R de Reynolds est par exemple supérieur au nombre critique de Reynolds Rc=2000 et, inversement, est dit "laminaire" lorsque le nombre R de Reynolds est environ inférieur au nombre critique de Reynolds Rc=2000.

On rappellera que le nombre R de Reynolds est défini par la formule :

$$R = Ux / v$$

dans laquelle "U" correspond à la vitesse moyenne d'écoulement, "x" une dimension linéaire de référence - tel que le diamètre "d" par exemple - et "v" au coefficient de viscosité cinématique du fluide qui est égal au quotient du coefficient de viscosité par la masse spécifique du fluide.

On a constaté qu'un tel écoulement turbulent de produit de stérilisation ou stérilisant à l'intérieur de la préforme conduit à la formation d'un ensemble de gouttelettes qui ne sont reparties de manière homogène sur la paroi interne de la préforme.

En effet, l'utilisation d'un pistolet de pulvérisation est caractérisé notamment par un débit important de produit stérilisant qui est obtenu en comprimant un gaz, tel que de l'air comprimé à des pressions d'environ 2 à 3 bars, de sorte qu'il produit un écoulement turbulent.

Or, l'écoulement turbulent conduit au dépôt non homogène de gouttelettes résiduelles de produit stérilisant sur les parois internes des préformes. De plus, les gouttelettes de produit stérilisant forment un excédent qui n'est pas entièrement vaporisé lors du chauffage.

Ces gouttelettes de produit stérilisant provoquent donc d'une part localement une attaque chimique du matériau de la préforme, généralement en polyéthylène téréphtalate (PET), et, d'autre part, lors du chauffage des préformes, produisent un effet loupe sur les rayonnements thermiques de chauffage, ce qui provoque l'apparition de taches sur les parois des bouteilles issues des préformes concernées.

L'apparition de telles taches sur les parois des bouteilles, est un défaut d'aspect du produit, qui est encore parfois appelé "aspect peau d'orange".

La présente invention vise notamment à remédier à ces inconvénients, plus particulièrement à permettre d'obtenir le degré de stérilité souhaitée au moins sur toute la paroi interne et le col de la préforme et de produire des bouteilles exemptes notamment de défaut d'aspect du type "peau d'orange".

Dans ce but, l'invention propose une installation selon la revendication 1.

Grâce à la projection du flux de produit stérilisant sous la forme d'un écoulement de type laminaire, par exemple à partir d'un mélange chaud de vapeur de produit tel que du peroxyde d'hydrogène ($H_2O_2$) et d'air, on obtient une répartition homogène du produit stérilisant sur la paroi interne de la préforme permettant par conséquent de supprimer le risque d'apparition de défaut d'aspect du type "peau d'orange".

Toutefois, suivant le profil des parois internes de chaque préforme, il n'est pas possible avec un écoulement de type laminaire d'atteindre de manière certaine le fond des préformes.

En effet, dès lors que l'on projette un flux laminaire de produit stérilisant dans une préforme, notamment de faible diamètre et de grande longueur, il se crée du fait même de ce type d'écoulement un bouchon d'air stagnant, dit de surpression, dans le fond de la préforme ce qui perturbe la condensation uniforme du produit stérilisant.

Grâce au décalage E de l'axe de projection de la buse par rapport à l'axe de la préforme, le flux laminaire de produit stérilisant se condense uniformément sur l'ensemble de la paroi interne de la préforme, y compris dans le fond.

Le problème de la formation d'un tel bouchon de surpression est ainsi avantageusement résolu et la totalité de la paroi interne de la préforme et du col sont parfaitement stérilisées par le flux laminaire de produit stérilisant.

Selon d'autres caractéristiques de l'invention :

- l'axe de projection est excentré d'au moins dix-neuf pourcents de la valeur du diamètre intérieur de l'ouverture délimitée par le col ;
- l'axe de projection est excentré au maximum de trente-deux pourcents de la valeur du diamètre intérieur de l'ouverture délimitée par le col ;
- la buse produit un flux de produit stérilisant globalement en forme de rideau vertical ;
- le dispositif de brumisation est pourvu de plusieurs buses qui sont globalement alignées longitudinalement, et les axes de projection des buses sont sensiblement parallèles ;
- le produit stérilisant est constitué d'un composé contenant du peroxyde d'hydrogène ou de peroxyde d'hydrogène vaporisé qui est activé par chauffage au-delà d'une température d'activation ;
- le peroxyde d'hydrogène est activé par chauffage à l'intérieur du four de l'unité de conditionnement thermique par chauffage de la préforme à une température supérieure à la température d'activation ;
- le peroxyde d'hydrogène est activé dans l'unité de traitement de traitement stérilisant par pulvérisation sur la préforme préalablement chauffée à une température supérieure à la température d'activation ;
- le peroxyde d'hydrogène est projeté à une température supérieure à cent six degrés Celsius, par exemple une température voisine de cent dix à cent vingt degrés Celsius.

D'autres caractéristiques et avantages de l'invention apparaîtront à la lecture de la description détaillée qui suit pour la compréhension de laquelle on se reportera aux dessins annexés dans lesquels :

- la figure 1 est un schéma qui représente un premier exemple de mise en oeuvre dans une installation produisant des bouteilles par soufflage et comportant une unité de stérilisation conforme aux enseignements de l'invention ;
- la figure 2 est une vue en coupe axiale suivant le plan de coupe 2-2 qui représente schématiquement une préforme dans l'unité de stérilisation de l'installation de la figure 3 ;
- la figure 3 est une vue de dessus qui représente une série de préformes dans l'unité de stérilisation ;
- la figure 4 est un schéma qui représente un deuxième exemple de mise en oeuvre dans une installation produisant des bouteilles par soufflage et comportant une unité de stérilisation conforme aux enseignements de l'invention.

Dans la suite de la description, des éléments similaires ou identiques seront désignés par les mêmes références.

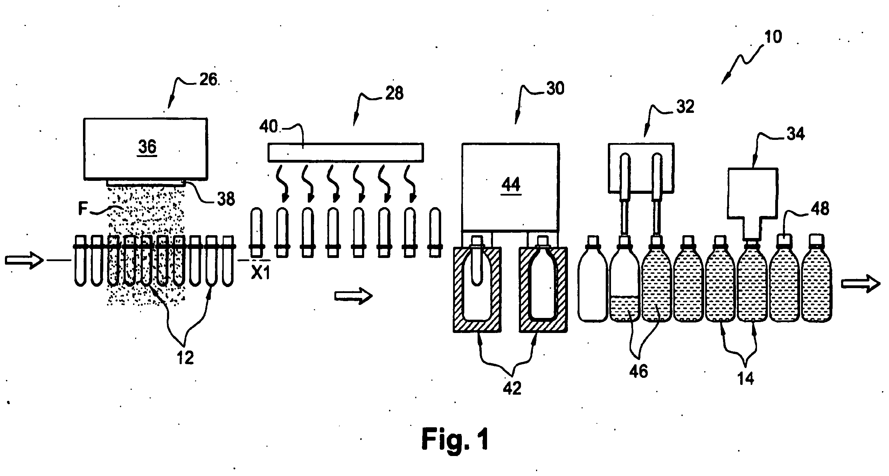

Sur la figure 1, on a représenté une installation 10 produisant des bouteilles 14 par soufflage à partir de préformes 12 en matière plastique.

Ce type d'installation 10 est utilisé, par exemple, pour la fabrication de bouteilles 14 en matière plastique telle que du polyéthylène téréphtalate (PET).

Chaque préforme 12 a globalement la forme d'un tube qui est fermé à une extrémité et dont l'autre extrémité possède déjà la forme définitive du col 16 de la bouteille 14.

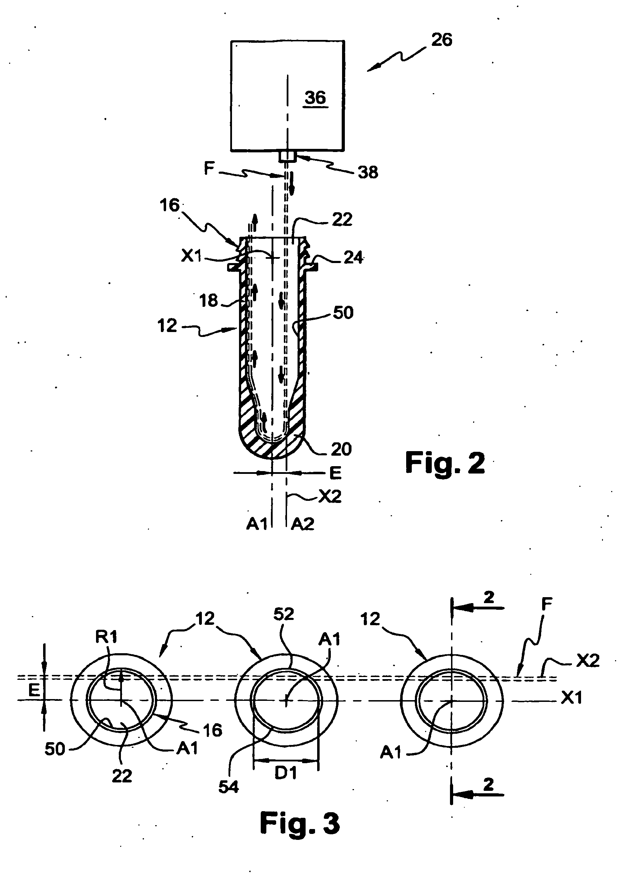

Sur la figure 2, une préforme 12 est représentée, à titre non limitatif, avec l'axe A1 de son corps 18 cylindrique qui s'étend verticalement.

L'axe A1 du corps 18 est confondu avec l'axe du col 16.

L'extrémité inférieure 20 de la préforme 12 est fermée, tandis que son extrémité supérieure forme le col 16 qui délimite une ouverture 22 et qui est pourvu ici d'une collerette 24 radiale externe.

Les préformes 12 sont ici réalisées au préalable, selon un procédé de moulage par injection.

Les préformes 12 sont convoyées à l'intérieur de l'installation 10 selon un flux continu qui circule de l'amont vers l'aval, c'est-à-dire de la gauche vers la droite en considérant la figure 1.

Dans le premier exemple représenté à la figure 1, l'installation 10 comporte, de l'amont vers l'aval, une unité 26, dite de traitement stérilisant, comportant de préférence des moyens de préparation du produit stérilisant, une unité de conditionnement thermique 28, et une unité de moulage 30.

Avantageusement, l'installation 10 comporte aussi, à la suite de l'unité de moulage 30, une unité de remplissage 32 et une unité de bouchage 34.

L'unité de traitement stérilisant 26 comporte un dispositif de brumisation 36 pourvu d'au moins une buse 38 qui projette, vers le col 16 de chaque préforme 12 en cours de traitement, un flux F de produit stérilisant de manière à produire des bouteilles 14 qui soient aseptisées ou stériles selon les applications.

L'unité de traitement stérilisant 26 sera décrite de manière plus détaillée par la suite, en rotation avec les caractéristiques de l'invention.

L'unité de conditionnement thermique 28 comporte au moins un four 40 qui chauffe les préformes 12 à une température appropriée au moulage.

L'unité de moulage 30 comporte au moins un moule 42 et au moins un dispositif de soufflage 44 qui soumet caque préforme 12 à une surpression interne de manière qu'elle prenne la forme de l'empreinte du moule 42 ce qui produit une bouteille 14.

L'unité de moulage 30 peut aussi comporter des moyens d'élongation (non représentés) qui étirent la préforme 12 vers le fond du moule 42, pendant l'opération de moulage.

L'unité de remplissage 32 injecte le produit 46 à conditionner dans les bouteilles 14 issues de l'unité de moulage 30, puis l'unité de bouchage 34 ferme hermétiquement les bouteilles stériles 14 remplies par exemple avec une capsule 48 appropriée, en variante un opercule et/ou un bouchon.

On décrira maintenant, de manière plus détaillée, un mode préféré de réalisation de l'unité de traitement stérilisant 26 selon l'invention.

De préférence, le produit stérilisant est constitué d'un composé contenant du peroxyde d'hydrogène ou de peroxyde d'hydrogène vaporisé ($H_2O_2$) qui est projeté vers les préformes 12 sous la forme d'un jet de gaz comportant du produit stérilisant à l'état de vapeur, avantageusement un jet de vapeur sèche.

A cet effet, les moyens (non représentés) de préparation du produit stérilisant de l'unité de traitement stérilisant 26 comportent notamment des moyens de chauffage (non représentés) du produit stérilisant et une source d'air (non représentée), avantageusement comprimé et/ou stérilisé par tout moyen approprié, qui est prévue pour propulser le produit stérilisant à travers la buse 38 du dispositif de brumisation 36.

L'air comprimé est avantageusement déshydraté et circule à faible vitesse selon un écoulement directif de manière à constituer un vecteur pour la vapeur de produit stérilisant.

La concentration de la vapeur en produit stérilisant est, par exemple, sensiblement égale à vingt-cinq pourcents.

A la sortie de la buse 38, la vapeur contenant le produit stérilisant atteint une température T donnée sensiblement supérieure à la température d'évaporation du produit stérilisant.

Dans le cas d'utilisation de peroxyde d'hydrogène ($H_2O_2$) la température T en sortie de buse est avantageusement supérieure à cent six degrés Celsius (106°C), de préférence comprise entre cent dix degrés Celsius (110°C) et cent vingt degrés Celsius (120°C).

Lorsque cette vapeur entre en contact avec les parois internes 50 des préformes 12, qui sont plus froides, le produit stérilisant se condense sous la forme de buée de sorte qu'un film de produit stérilisant se dépose sur l'ensemble de la préforme 12, en particulier sur les parois internes 50 des préformes 12 qui s'embuent ainsi d'un film de produit stérilisant.

Avantageusement, le produit stérile se dépose donc par condensation suivant un film sensiblement uniforme qui permet, par rapport à l'état de la technique, d'éliminer tout risque d'apparition de taches et d'aspect "peau d'orange".

Les préformes 12 passent alors dans l'unité de conditionnement thermique 28 comportant le four 40 de chauffage pour réchauffer les préformes 12 à une température supérieure ou égale à la température de moulage afin de pouvoir réaliser ensuite l'opération de soufflage.

Bien entendu, la température de moulage dépend du type de préforme 12 qui est fonction des applications, à titre d'exemple la température est par exemple comprise entre quatre vingt quinze degrés Celsius (95°C) et cent trente cinq degrés Celsius (135°C).

Or, la stérilisation est obtenue en portant la préforme 12 recouverte du film de buée de produit stérilisant au-delà d'une température, dite température d'activation.

La température d'activation du peroxyde d'hydrogène ($H_2O_2$) est par exemple à partir d'environ soixante dix degrés Celsius (70°C), c'est-à-dire ici une température inférieure à la température de moulage.

Avantageusement, le passage des préformes 12 dans l'unité de conditionnement thermique 28 provoque donc l'activation du produit stérilisant par chauffage, ce qui a un effet bactéricide immédiat sur les parois internes 50 des préformes 12.

De manière connue (non représentée), un tel four 40 de chauffage pour préformes comporte un tunnel longitudinal de chauffage le long duquel les préformes 12 sont transportées par un dispositif de transport entre une première extrémité du tunnel où les préformes 12 pénètrent généralement froides avant de ressortir par la seconde extrémité du tunnel chauffées ou réchauffées, prêtes pour l'opération de soufflage.

Pour assurer le chauffage d'une préforme 12 en profondeur, c'est-à-dire tant de l'extrémité inférieure 20 formant fond que de la paroi cylindrique du corps 18, les préformes 12 sont généralement mises en rotation sur elles-mêmes pendant leur circulation dans le four 40 au moyen d'un dispositif de transport comportant des moyens de préhension par exemple du type de ceux décrits dans le document WO-A-00/48819.

Par ailleurs, une paroi du tunnel est équipée de moyens de chauffage à rayonnement tandis que l'autre paroi est munie d'orifices d'aération pour permettre le passage d'air soufflé afin de favoriser un chauffage homogène dans toute l'épaisseur de la préforme 12 sans surchauffer la couche de matière superficielle.

En effet, l'air soufflé permet d'évacuer la chaleur de convection provoquée par les moyens de chauffage pour favoriser la pénétration du rayonnement qu'ils produisent dans l'épaisseur de la matière constituant la préforme 12.

Pour plus de détails sur de tels fours de chauffage 40 de préformes, on se reportera par exemple aux documents EP-A-0.620.099 ou EP-A-0.564.354.

Cependant dans le cas d'une installation 10 telle que représentée à la figure 1, l'air soufflé sur les préformes 12 dans le four 40 provoque l'élimination de tout ou partie du film de buée de produit stérile préalablement déposé sur l'extérieur de la préforme 12 par condensation dans l'unité de traitement stérile 26.

On obtient donc, à la sortie du four 40, des préformes 12 qui sont principalement stériles à l'intérieur.

Grâce à une installation 10 selon le premier exemple de mise en oeuvre, on obtient une réduction logarithmique du nombre de germes de l'ordre de 3D, ou encore 3 Log équivalent à 1000 unités ($10^3$).

De manière connue, la quantité de germes est par exemple dénombrée par comptage après des opérations de lavage, de filtration et de mise en culture.

Avantageusement, le four 40 comporte des moyens de protection notamment pour limiter la corrosion des parties ou pièces exposées au produit stérile ainsi soufflée à l'intérieur du tunnel.

Selon une variante de réalisation (non représentée) de l'installation 10, une enceinte de confinement stérile peut être prévue pour permettre un traitement des préformes 12 et des bouteilles 14 complètement aseptique.

Selon le mode de réalisation représenté ici, notamment sur la figure 3, les préformes 12 défilent dans l'unité de traitement stérilisant 26 en étant alignées, en position verticale, suivant une direction horizontale longitudinale, dite direction X1 de défilement, le col 16 vers le haut.

La direction X1 de défilement passe par les axes A1 des préformes 12 en cours de traitement.

Conformément aux enseignements de l'invention, l'axe A2 moyen de projection de la buse 38 est globalement parallèle à l'axe A1 de chaque préforme 12 en cours de traitement et il (A2) est excentré radialement, par rapport à l'axe A1 de la préforme 12, d'une valeur de décalage E déterminée et le flux F de produit stérilisant est projeté à l'intérieur de la préforme 12 sous la forme d'un écoulement de type laminaire.

De préférence, l'axe A2 moyen de projection, qui est ici vertical, est excentré suivant un rayon intérieur R1 du col 16 qui est orthogonal à la direction X1 de défilement.

La forme de la buse 38 permet de projeter, vers le bas, un flux F de produit stérilisant globalement sous la forme d'un flux laminaire, c'est-à-dire ici sous la forme d'un rideau vertical et de préférence longitudinal.

A cet effet la buse 38 comporte avantageusement une fente longitudinale ou circulaire, en variante par exemple un trou globalement circulaire de projection du flux F.

Le flux F laminaire s'étend ici globalement suivant un plan vertical longitudinal, dit plan de projection X2, qui est décalé radialement, par rapport à la direction X1 de défilement, d'une distance égale au décalage E.

Le flux F de produit stérilisant admet ici une infinité d'axes A2 moyens de projection qui s'étendent verticalement dans le plan de projection X2.

De préférence, la valeur de décalage E est comprise entre une valeur minimale Emin sensiblement égale à dix-neuf pourcents du diamètre intérieur D1 du col 16 de chaque préforme 12 et une valeur maximale Emax sensiblement égale à trente-deux pourcents du diamètre intérieur D1 du col 16.

Selon un mode de réalisation avantageux, la valeur de décalage E est choisie fixe et sensiblement égale à huit millimètres, de sorte qu'elle convient à des modèles de préformes 12 possédant des diamètres intérieurs D1 compris environ entre vingt-cinq et quarante deux millimètres.

Grâce à l'agencement de la buse 38 selon l'invention, le flux F de produit stérilisant est sensiblement affleurant avec un premier secteur 52 de la paroi internes 50 de chaque préforme 12, de sorte que le flux F de produit stérilisant lèche ledit secteur 52 de la paroi interne 50.

En arrivant à l'extrémité inférieure 20 de la préforme 12, le flux F de produit stérilisant glisse sur le fond de la préforme 12 et remonte le long d'un second secteur 54 de paroi interne 50, diamétralement opposé au premier 52.

Ainsi, le flux F de produit stérilisant balaye globalement l'ensemble de la paroi interne 50 de chaque préforme 12, selon un écoulement de type laminaire.

L'agencement selon l'invention permet notamment d'empêcher la création d'un bouchon de surpression, dans le fond des préformes 12, qui empêcherait le produit stérilisant d'atteindre le fond.

En particulier, la vitesse de propulsion du produit stérilisant, à la sortie de la buse 38, est suffisamment faible pour obtenir un écoulement de type laminaire, par exemple de l'ordre de 0,3 à 0,5 m/s avec une buse d'une longueur d'environ 35 mm et de diamètre calibré d'environ 3 mm.

On note que le flux F de produit stérilisant crée un brouillard de produit stérilisant qui se diffuse tout autour du flux F, ce qui permet au produit stérilisant de se déposer notamment sur l'ensemble de la paroi interne 50 de chaque préforme 12.

De plus, ce brouillard se dépose aussi sur la paroi extérieure du col 16, ce qui permet de d'aseptiser ou de stériliser simultanément la paroi interne 50 et le col 16 de chaque préforme 12.

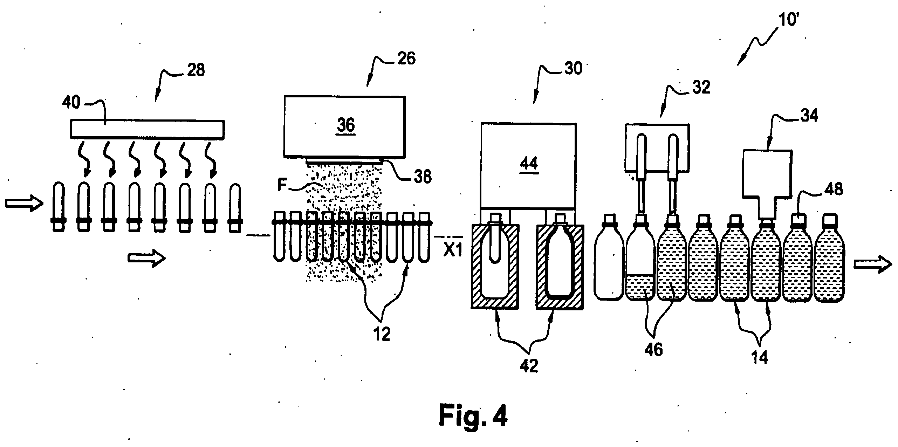

La figure 4 représente un deuxième exemple de mise en oeuvre de l'invention dans une installation 10' produisant des bouteilles stériles par soufflage qui; similaire à celle de la figure 1, sera ci-après décrite par comparaison.

L'installation 10' selon la figure 4 diffère essentiellement par l'inversion opérée entre l'unité de conditionnement thermique 28 et l'unité de traitement stérilisant 26.

Ainsi, l'installation 10' comporte, respectivement de l'amont vers l'aval, une unité de conditionnement thermique 28, une unité de traitement stérilisant 26 et une unité de moulage 30.

Les unités 26, 28 et 30 sont ici analogues à celles de la figure 1.

Les préformes 12 sont donc d'abord chauffées par l'unité de conditionnement thermique 28 de manière à être portées à une température supérieure ou égale à la température de moulage, par exemple comprise entre quatre vingt quinze degrés Celsius (95°C) et cent trente cinq degrés Celsius (135°C).

Puis les préformes 12 ainsi chauffées passent dans l'unité de traitement stérilisant 26 pour y être stérilisées.

Cependant comme les préformes 12 sont à une température supérieure à la température d'activation, par exemple de l'ordre d'environ soixante dix degrés Celsius (70°C) pour le peroxyde d'hydrogène ($H_2O_2$), il ne se produit pas de condensation comme précédemment et donc de dépôt d'un film uniforme de buée de produit stérilisant.

En effet, le produit stérilisant est instantanément activé et s'évapore au contact de la préforme chauffée 12 en produisant dès lors un effet bactéricide analogue sur l'ensemble de la préforme 12, c'est-à-dire tant sur la paroi interne 50 que sur les parties externes de la préforme 12.

Grâce à l'installation 10' selon la figure 4, on stérilise donc entièrement les préformes 12 et on obtient des degrés de stérilisation de l'ordre de 6 Log, c'est-à-dire supérieur à ceux obtenu avec l'installation 10 de la figure 1.

Avantageusement, le four 40 de l'unité de conditionnement thermique 28 est un four conventionnel qui, se trouvant en amont de l'unité de traitement stérilisant, n'est pas comme précédemment exposés à des risques de corrosion par le produit stérilisant et ne nécessite donc pas de moyens de protection complémentaires particuliers.

Les préformes 12 parcourant l'installation 10 ou 10', notamment l'unité de traitement stérilisant 26, sont ici orientées verticalement avec le col 16 vers le haut, c'est-à-dire en position dite "col en haut".

En variante, les préformés 12 sont orientées verticalement l'unité de traitement stérilisant 26 avec le col 16 vers le bas, soit "col en bas", les préformes 12 étant susceptibles de changer d'orientation verticale au sein de l'installation 10, 10', en particulier d'une unité à l'autre.

L'invention a été décrite avec un dispositif de brumisàtion 36 comportant avantageusement une buse 38 en forme de fente longitudinale ou circulaire. Bien entendu, selon des variantes de réalisation (non représentées), le flux F de produit stérilisant peut être réalisé par plusieurs buses 38 tubulaires alignées suivant le plan de projection X2.

On note que les installations 10 et 10' ont été représentées avec des unités de traitement 26, 28, 30, 32, 34 alignées, à titre d'illustration, mais ces unités de traitement peuvent être agencées selon une configuration différente.

Certaines unités de traitement 26, 28, 30, 32, 34 peuvent mettre en oeuvre des dispositifs tournant tels que des carrousels (non représentés).

## Revendications

**1.** Installation (10, 10') produisant des bouteilles (14) stériles par soufflage à partir de préformes (12) en matière plastique, du type dans lequel les préformes (12) sont convoyées à l'intérieur de l'installation (10, 10') selon un flux continu qui circule de l'amont vers l'aval, et du type comportant :

- une unité de traitement stérilisant (26) comportant un dispositif de brumisation (36) pourvu d'au moins une buse (38) qui projette, vers le col (16) de chaque préforme (12) en cours de traitement, un flux (F) de produit stérilisant, sous forme d'un jet, en vue de stériliser au moins les parois internes (50) de la préforme (12),
- une unité de conditionnement thermique (28) comportant au moins un four (40) qui chauffe les préformes (12),
- une unité de moulage (30) comportant au moins un moule (42) et au moins un dispositif de soufflage (44) qui soumet chaque préforme (12) à une surpression interne de manière qu'elle prenne la forme de l'empreinte du moule (42) ce qui produit une bouteille (14),

**caractérisée en ce que** l'axe (A2) moyen de projection de la buse (38) est globalement parallèle à l'axe (A1) de la préforme (12) en cours de traitement et excentré radialement, par rapport à l'axe (A1) de la préforme (12) d'une valeur de décalage (E) déterminée,

en ce que le flux (F) de produit stérilisant est projeté à l'intérieur de la préforme (12) sous la forme d'un écoulement de type laminaire,

en ce que dans l'unité de traitement stérilisant (26), les préformes (12) sont globalement alignées suivant une direction (X1) longitudinale de défilement, et sont disposées en position debout, parallèlement les unes à côté des autres,

et en ce que l'axe (A2) de projection de la buse (38) est décalé radialement suivant une direction sensiblement orthogonale, par rapport à la direction (X1) de défilement.

2. Installation (10, 10') selon la revendication 1, caractérisée en ce que l'axe (A2) de projection est excentré d'au moins dix-neuf pourcents de la valeur du diamètre intérieur (D1) de l'ouverture (22) délimitée par le col (16).

3. Installation (10, 10') selon l'une des revendications 1 ou 2, caractérisée en ce que l'axe (A2) de projection est excentré au maximum de trente-deux pourcents de la valeur du diamètre intérieur (D1) de l'ouverture (22) délimitée par le col (16).

4. Installation (10, 10') selon la revendication 1, caractérisée en ce que la buse (38) produit un flux (F) de produit stérilisant globalement en forme de rideau vertical.

5. Installation (10, 10') selon l'une des revendications 1 ou 4 , caractérisée en ce que le dispositif de brumisation (36) est pourvu de plusieurs buses (38) qui sont globalement alignées longitudinalement, et en ce que les axes (A2) de projection des buses (38) sont sensiblement parallèles.

6. Installation (10, 10') selon l'une quelconque des revendications précédentes, caractérisée en ce que le produit stérilisant est constitué d'un composé contenant du peroxyde d'hydrogène ou de peroxyde d'hydrogène vaporisé qui est activé par chauffage au-delà d'une température d'activation.

7. Installation (10) selon la revendication 6, caractérisée en ce que le peroxyde d'hydrogène est activé par chauffage à l'intérieur du four (40) de l'unité de conditionnement thermique (28) par chauffage de la préforme (12) à une température supérieure à la température d'activation.

8. Installation (10') selon la revendication 6, caractérisée en ce que le peroxyde d'hydrogène est activé dans l'unité de traitement de traitement stérilisant (26) par pulvérisation sur la préforme (12) préalablement chauffée à une température supérieure à la température d'activation.

9. Installation (10, 10') selon l'une des revendications 6 ou 8 , caractérisée en ce que le peroxyde d'hydrogène est projeté à une température supérieure à cent six degrés Celsius.

## Patentansprüche

1. Anlage (10, 10') zur Herstellung steriler Flaschen (14) durch Blasformen von Vorformen (12) aus Kunststoff, wobei die Vorformen (12) entsprechend einem kontinuierlichen Strom, der von oben nach unten zirkuliert, in das Innere der Anlage (10, 10') transportiert werden, die folgendes aufweist:

- eine Sterilisationseinheit (26), welche eine Vorrichtung zur Zerstäubung (36) besitzt, die mit mindestes einer Düse (38) versehen ist, welche im Verlaufe der Sterilisierung einen Strom (F) des Sterilisationsproduktes in Form eines Strahls in Richtung des Halses (16) jeder Vorform (12) sprüht, um mindestens die Innenwände (50) der Vorform (12) zu sterilisieren,
- eine Einrichtung zur thermischen Aufbereitung (28) mit mindestens einem Ofen (40), der die Vorformen (12) aufheizt,
- eine Einrichtung zum Formen (30), die mindestens eine Form (42) und mindestens eine Blasvorrichtung (44) besitzt, welche jede Vorform (12) einem inneren Überdruck aussetzt, so dass sie die Form des Hohlraums der Form (42) annimmt, so dass eine Flasche (14) hergestellt wird,

dadurch gekennzeichnet, dass die Mittelachse (A2) der Düse (38) zum Sprühen im allgemeinen parallel zu der Achse (A1) der zu sterilisierenden Vorform und um einen festgelegten Versatzwert (E) radial exzentrisch zu der Achse (A1) der Vorform (12) verläuft, und dass der Strom des Sterilisationsproduktes in Form einer Laminarströmung in die Vorform (12) hinein gesprüht wird,

dass die Vorformen (12) in der Sterilisationseinheit (26) im allgemeinen entlang einer längs verlaufenden Durchlaufrichtung (X1) ausgerichtet und in einer aufrecht stehenden Position parallel nebeneinander angeordnet sind,

und dass die Sprühachse der Düse (38) entlang einer im wesentlichen orthogonalen Richtung zu der Durchlaufrichtung (X1) radial versetzt ist.

2. Anlage (10, 10') nach Anspruch 1, dadurch gekennzeichnet, dass die Sprühachse (A2) um mindestens neunzehn Prozent vom Wert des Innendurchmessers (D1) der Öffnung (22), die durch den Hals (16) begrenzt ist, exzentrisch angeordnet ist.

3. Anlage (10, 10') nach einem der Ansprüche 1 oder 2, **dadurch gekennzeichnet, dass** die Sprühachse (A2) um höchsten zweiunddreißig Prozent vom Wert des Innendurchmessers (D1) der Öffnung (22), die durch den Hals (16) begrenzt ist, exzentrisch angeordnet ist.

4. Anlage (10, 10') nach Anspruch 1, **dadurch gekennzeichnet, dass** die Düse (38) einen Strom (F) des Sterilisationsproduktes erzeugt, der im allgemeinen die Form eines vertikalen Vorhanges hat.

5. Anlage (10, 10') nach einem der vorhergehenden Ansprüche 1 oder 4, **dadurch gekennzeichnet, dass** die Vorrichtung zur Zerstäubung (36) mit mehreren Düsen (38) versehen ist, die im allgemeinen in Längsrichtung ausgerichtet sind, und dass die Sprühachsen (A2) der Düsen im wesentlich parallel verlaufen.

6. Anlage (10, 10') nach dem vorhergehenden Anspruch, **dadurch gekennzeichnet, dass** das Sterilisationsprodukt aus einer Verbindung hergestellt ist, die Wasserstoffperoxid oder verdampftes Wasserstoffperoxid enthält, das durch Erhitzen über eine Aktivierungstemperatur hinaus aktiviert wird.

7. Anlage (10) nach Anspruch 6, **dadurch gekennzeichnet, dass** das Wasserstoffperoxid durch Erhitzen im Inneren des Ofens (40) der Einrichtung zur thermischen Aufbereitung (28) aktiviert wird, indem die Vorform (12) auf eine Temperatur oberhalb der Aktivierungstemperatur erhitzt wird.

8. Anlage (10') nach Anspruch 6, **dadurch gekennzeichnet, dass** das Wasserstoffperoxid in der Sterilisationseinheit (26) durch Zerstäubung über der Vorform (12), die zuvor auf eine Temperatur oberhalb der Aktivierungstemperatur erhitzt wurde, aktiviert wird.

9. Anlage (10, 10') nach einem der Ansprüche 6 oder 8, **dadurch gekennzeichnet, dass** das Wasserstoffperoxid bei einer Temperatur oberhalb von einhundertsechs Grad Celsius zerstäubt wird.

**Claims**

1. An installation (10, 10') producing sterile bottles (14) by blow molding starting from plastic preforms (12), of the type where the preforms (12) are conveyed to the interior of the installation (10, 10') in a continuous stream which flows from upstream to downstream, and of the type including:

> - a sterilizing treatment unit (26) including an atomizing device (36) provided with at least one nozzle (38) which sprays a stream (F) of sterilizing product in the form of a jet towards the neck (16) of each preform (12) during treatment, with the aim of sterilizing at least the internal walls (50) of the preform (12),
> - a thermal conditioning unit (28), including at least one oven (40) which heats the preforms (12),
> - a molding unit (30) including at least one mold (42) and at least one blow-molding device (44) which subjects each preform (12) to internal overpressure in such a way that it assumes the shape of the mold cavity (42), producing a bottle (14),

**characterized in that** the mean axis (A2) of spraying of the nozzle (38) is generally parallel to the axis (A1) of the preform (12) during treatment and is radially off-center, with regard to the axis (A1) of the preform (12), of a determined offset value (E), and **in that** the form in which the stream (F) of sterilizing product is sprayed at the interior of the preform (12) is that of laminar flow,

**in that,** in the sterilizing treatment unit (26), the preforms (12) are generally aligned in a longitudinal running direction (X1), and are arranged upright parallel to, and alongside, one another,

and **in that** the axis (A2) of spraying of the nozzle (38) is radially offset in an approximately orthogonal direction with regard to the running direction (X1).

2. The installation (10, 10') as claimed in claim 1, **characterized in that** the axis (A2) of spraying is off center to the extent of at least nineteen percent of the value of the internal diameter (D1) of the opening (22) delimited by the neck (16).

3. The installation (10, 10') as claimed in claim 1 or 2, **characterized in that** the axis (A2) of spraying is off-center to the extent of at most thirty-two percent of the value of the internal diameter (D1) of the opening (22) delimited by the neck (16).

4. The installation (10, 10') as claimed in the preceding claim, **characterized in that** the form of the stream (F) of sterilizing product produced by the nozzle (38) is generally that of a vertical curtain.

5. The installation (10, 10') as claimed in claim 1 or 4, **characterized in that** the atomizing device (36) is provided with a plurality of nozzles (38) which are generally aligned longitudinally, and **in that** the axes (A2) of spraying of the nozzles (38) are approximately parallel.

6. The installation (10, 10') as claimed in any of the preceding claims, **characterized in that** the sterilizing product is made from a compound containing hydrogen peroxide, or is made from vaporized hydrogen peroxide, which is activated by heating beyond an activation temperature.

7. The installation (10) as claimed in claim 6, **characterized in that** the hydrogen peroxide is activated by heating in the interior of the oven (40) of the thermal conditioning unit (28) by heating of the preform (12) to a temperature above the activation temperature.

8. The installation (10') as claimed in claim 6, **characterized in that** the hydrogen peroxide is activated in the sterilizing treatment unit (26) by spraying onto the preform (12) previously heated to a temperature above the activation temperature.

9. The installation (10, 10') as claimed in claim 6 or 8, **characterized in that** the hydrogen peroxide is sprayed at a temperature above one hundred and six degrees Celsius.

Fig. 1

EP 1 896 329 B2

Fig. 2

Fig. 3

11

Fig. 4

**EP 1 896 329 B2**

**Documents brevets cités dans la description**

- US 20010010145 A
- WO 9903667 A2
- WO 0048819 A
- EP 0620099 A
- EP 0564354 A